# EUROPEAN PATENT APPLICATION

(11) **EP 1 430 904 A1**
(43) Date of publication of application: **23.06.2004**
(21) Application number: 02765361.7
(22) Date of filing: 28.08.2002
(51) Int. Cl.: A61K 39/00, A61K 38/00, A61P 35/00, A61P 37/08

(54) **DESENSITIZERS**

(30) Priority: 29.08.2001 JP 2001260046
(71) Applicant: Itoh, Kyogo, Miyaki-gun, Saga 841-0205 (JP)
(72) Inventor: ITOH, Kyogo, Miyaki-gun, Saga 841-0205 (JP); YAMADA, Akira, Ogori-shi, Fukuoka 838-0103 (JP)
(74) Representative: Krauss, Jan B., Dr.
(86) International application number: PCT/JP2002/008641
(87) International publication number: WO 2003/020306

(57) **Abstract**

An agent is described for suppressing an allergic reaction, comprising a peptide which is derived from the same antigenic substance as the antigenic substance eliciting the allergic reaction, contains an epitope different from an epitope participating in elicitation of the allergic reaction and yet does not elicit the allergic reaction. Also described is a medicament for preventing and/or treating type I allergic diseases or a pharmaceutical composition comprising the agent for suppressing an allergic reaction. A method for suppressing allergic reactions, a method of desensitization, and a method for preventing and/or treating type I allergic diseases with use of the same are further described. Also described is a cancer vaccine comprising a tumor rejection antigen-derived peptide which can induce a cytotoxic T lymphocyte but induces an allergic reaction and another peptide derived from the same antigen as the above peptide and has an epitope different from an epitope of the above peptide and exhibits an effect of suppressing the allergic reaction. A medicament is further described for treating cancer comprising the cancer vaccine, and a method for treating cancer with the use of the cancer vaccine is also described.

## Description

### FIELD OF THE INVENTION

The present invention relates generally to an agent for suppressing an allergic reaction or an agent of desensitization, and more particularly, to an agent for suppressing an allergic reaction or an agent of desensitization, that comprises a major histocompatibility complex (MHC) class I-restricted peptide.

### BACKGROUND OF THE INVENTION

'An allergic reaction' means a so-called hypersensitivity reaction in which an immune reaction to an antigen occurs excessively or in an improper manner to damage a tissue. An allergic reaction is classified into four types, i.e., type-I, type-II, type-III, and type-IV. Among these, type-I allergy, which is also called 'immediate type allergy' or 'immediate type hypersensitivity', is elicited by an antigen-specific immunoglobulin E antibody (which may be abbreviated as 'IgE' hereafter).

Type I allergy is generally considered to be a hypersensitivity reaction to foreign antigens, and is observed in a living body, which was stimulated with an antigen, within several minutes to several hours after the second stimulation with the same antigen. Antigenic substances that cause an allergic reaction are called 'allergens', which include plant pollens, fungi, mites, animal smuts, house dusts, plant components, drugs, insect toxins, and the like. In a living body that was stimulated with an allergen, antigen-specific IgE is produced, which binds to a mast cell and basophil having IgE receptors on their surface. When the living body comes in contact again with the same allergen, IgE molecules bound to the surface of these cells are cross-linked by the allergen, resulting in release of various chemical mediators such as histamine from the cells. These chemical mediators cause an allergic symptom, called 'anaphylaxis', which is accompanied by peripheral circulatory failure such as inflammation, angioedema, suffusion, systemic urticaria, constriction of bronchial smooth muscle, and/or depression of blood pressure. Such mechanisms are involved in allergic diseases, for example, allergic rhinitis, allergic asthma, atopic dermatitis, and so on, which has become a problem in recent years.

Hyposensitization or desensitization is known as a method for treating type-I allergy, in which a whole allergen is administered stepwise little by little to reduce sensitivity to an etiologic allergen. Such therapy is applied particularly to inhalant allergens such as pollens, fungi, mites, animal smuts, and house dusts. It is considered that repeated administration of a small amount of allergen as described above induces production of a large amount of immunoglobulin G antibody (which may be abbreviated as 'IgG' hereafter), whereupon the IgG binds to the allergen resulting in inhibition of the binding of IgE to the allergen, and/or the production of antigen-specific IgE is inhibited, hence an allergic reaction is suppressed. However, desensitization in which a whole allergen is administered brings a load to a patient even when a small amount of allergen is administered. Thus, a new therapy effective for allergic diseases is desired.

An immune reaction is originally a host defense mechanism against foreign antigens and is an essential reaction for maintaining a normal and healthy condition. However, the immune reaction to a self-antigen is sometimes elicited, resulting in autoimmune diseases such as rheumatoid arthritis and insulin-dependent diabetes mellitus. A mechanism of tissue-damage in autoimmune diseases is basically the same as that of the immune reaction in allergy, and it has been reported that type-II, type-III, and type-IV allergies could be involved in autoimmune diseases. However, it has not been considered so far that type-I allergy plays an important role in autoimmune diseases.

Recently, anaphylactic shock against a self-peptide in mice with experimental allergic encephalomyelitis (EAE) has been reported [Nature Immunology (2001) 2, 193]. The EAE mouse is an animal model of human multiple sclerosis and of autoimmune disease mediated by CD4⁺ T helper 1 (Th1) cells. EAE can be induced by immunizing SJL mice with a self-peptide such as myelin proteolipid protein (PLP) peptide 139-151 (PLPp139-151) together with complete Freund adjuvant (CFA). Namely, immunizing a mouse with an emulsion prepared from PLPp139-151 with CFA or incomplete Freund adjuvant (IFA) followed by injection of PLPp139-151, would induce an anaphylactic shock in the mouse. In this report, existence of self-peptide-specific IgE was not verified. However, anaphylactic shock is phenotypically IgE-mediated type-I allergy. These results would suggest that type-I allergy is involved in immune reaction to self-antigens.

In addition, it has been reported that an IgE-mediated reaction to self-antigens is involved in serious atopic diseases [Susanne, N., FASEB J. (1998) 12: 1559-1569; Ulrich, A., Int. Arch. Allergy Immunol. (1999) 118: 193-196].

### SUMMARY OF THE INVENTION

The inventors carried out hundreds of examinations exhaustively in order to regulate a type-I allergic reaction, for example, a type-I allergic reaction to a self-antigen, and they found that repeated administration of a non-allergic peptide permits suppression of an antigen-specific IgE reaction and a type-I allergy, wherein the non-allergic peptide comprises a partial sequence being different from an epitope peptide capable of causing an allergic reaction (which will be called 'allergic peptide' hereafter) in an amino acid sequence of an antigenic substance capable of causing the allergic reaction, consequently the present invention has been achieved.

An embodiment of the present invention can be an agent for suppressing an allergic reaction that comprises a peptide which is derived from the same antigenic substance as the antigenic substance eliciting the allergic reaction, and contains an epitope different from an epitope participating in elicitation of the allergic reaction and yet does not elicit the allergic reaction.

Another embodiment of the present invention can be an agent for suppressing an allergic reaction that comprises a peptide which is derived from the same antigenic substance as the antigenic substance from which a peptide eliciting the allergic reaction is derived, and contains an epitope different from an epitope participating in elicitation of the allergic reaction and yet does not elicit the allergic reaction.

A further embodiment of the present invention can be an agent for suppressing an allergic reaction that comprises a peptide which is derived from the same self-antigen as the self antigen eliciting the allergic reaction, and contains an epitope different from an epitope participating in elicitation of the allergic reaction and yet does not elicit the allergic reaction.

Still another embodiment of the present invention can be an agent for suppressing an allergic reaction that comprises a peptide which is derived from the same self-antigen as the self-antigen from which a peptide eliciting the allergic reaction is derived, and contains an epitope different from an epitope participating in elicitation of the allergic reaction and yet does not elicit the allergic reaction.

Yet another embodiment of the present invention can be the above-described agent for suppressing an allergic reaction, wherein the peptide which contains an epitope different from an epitope participating in elicitation of the allergic reaction and that does not elicit the allergic reaction is an MHC class I-restricted peptide.

Another embodiment of the present invention can be an agent for suppressing an allergic reaction elicited by a tumor rejection antigen-derived peptide capable of inducing a cytotoxic T lymphocyte, wherein the agent comprises a peptide which is derived from the same tumor rejection antigen as the tumor rejection antigen-derived peptide, and contains an epitope different from an epitope participating in elicitation of the allergic reaction and yet does not elicit the allergic reaction.

A further embodiment of the present invention can be the above-described agent for suppressing an allergic reaction, wherein the tumor rejection antigen is one or more selected from a group consisting of cyclophilin B, ART4, SART2 and SART3.

Still another embodiment of the present invention can be an agent for suppressing an allergic reaction elicited by the peptide of SEQ ID NO: 1 shown in the sequence listing, which is derived from cyclophilin B, wherein the agent comprises a peptide of SEQ ID NO: 2 or of SEQ ID NO: 9 shown in the sequence listing, which is derived from cyclophilin B.

Yet another embodiment of the present invention can be an agent for suppressing an allergic reaction elicited by the peptide of SEQ ID NO: 3 shown in the sequence listing, which is derived from ART4, wherein the agent comprises a peptide of SEQ ID NO: 4 shown in the sequence listing, which is derived from ART4.

Another embodiment of the present invention can be an agent for suppressing an allergic reaction elicited by the peptide of SEQ ID NO: 6 shown in the sequence listing which is derived from SART2, wherein the agent comprises a peptide of SEQ ID NO: 5 shown in the sequence listing which is derived from SART2.

A further embodiment of the present invention can be an agent for suppressing an allergic reaction elicited by the peptide of SEQ ID NO: 7 shown in the sequence listing which is derived from SART3, wherein the agent comprises a peptide of SEQ ID NO: 8 shown in the sequence listing which is derived from SART3.

Still another embodiment of the present invention can be an agent for preventing and/or treating type-I allergic diseases, comprising the above-described agent for suppressing an allergic reaction.

Yet another embodiment of the present invention can be a method for preventing and/or treating type-I allergic diseases, comprising using the above-described agent for suppressing an allergic reaction.

Another embodiment of the present invention can be a method for suppressing an allergy, comprising using the above-described agent for suppressing an allergic reaction.

A further embodiment of the present invention can be a method for reducing production of immunoglobulin E antibody, comprising using the above-described agent for suppressing an allergic reaction.

Still another embodiment of the present invention can be a method of desensitization, comprising using the above-described agent for suppressing an allergic reaction.

Yet another embodiment of the present invention can be a method for producing the above-described agent for suppressing an allergic reaction.

Another embodiment of the present invention can be a pharmaceutical composition that comprises a combination of a protein and/or peptide capable of eliciting an allergic reaction, and a peptide which is derived from the same antigenic substance as the protein and/or peptide and contains an epitope different from an epitope participating in elicitation of the allergic reaction and yet does not elicit the allergic reaction.

A further embodiment of the present invention can be a cancer vaccine comprising a tumor rejection antigen-derived peptide having an activity that induces a cytotoxic T lymphocyte and elicits an allergic reaction, and a peptide which is derived from the same antigenic substance as the tumor rejection antigen-derived peptide and contains an epitope different from an epitope contained in tumor rejection antigen-derived peptide participating in elicitation of the allergic reaction and yet does not elicit the allergic reaction.

Still another embodiment of the present invention can be the above-described cancer vaccine, comprising a peptide of SEQ ID NO: 1 shown in the sequence listing, and a peptide of SEQ ID NO: 2 or a peptide of SEQ ID NO: 9 shown in the sequence listing, wherein the peptides are derived from cyclophilin B.

Yet another embodiment of the present invention can be the above-described cancer vaccine, comprising a peptide of SEQ ID NO: 3 shown in the sequence listing, and a peptide of SEQ ID NO: 4 shown in the sequence listing, wherein the peptides are derived from ART4.

Another embodiment of the present invention can be the above-described cancer vaccine, comprising a peptide of SEQ ID NO: 6 shown in the sequence listing, and a peptide of SEQ ID NO: 5 shown in the sequence listing, wherein the peptides are derived from SART2.

A further embodiment of the present invention can be the above-described cancer vaccine, comprising a peptide of SEQ ID NO: 7 shown in the sequence listing, and a peptide of SEQ ID NO: 8 shown in the sequence listing, wherein the peptides are derived from SART3.

Still another embodiment of the present invention can be an agent for treating cancer, comprising the above-described cancer vaccine.

Yet another embodiment of the present invention can be a method for preventing and/or treating cancer, comprising using the above-described cancer vaccine.

Another embodiment of the present invention can be a method for producing the above-described pharmaceutical composition.

### BRIEF DESCRIPTION OF THE DRAWINGS

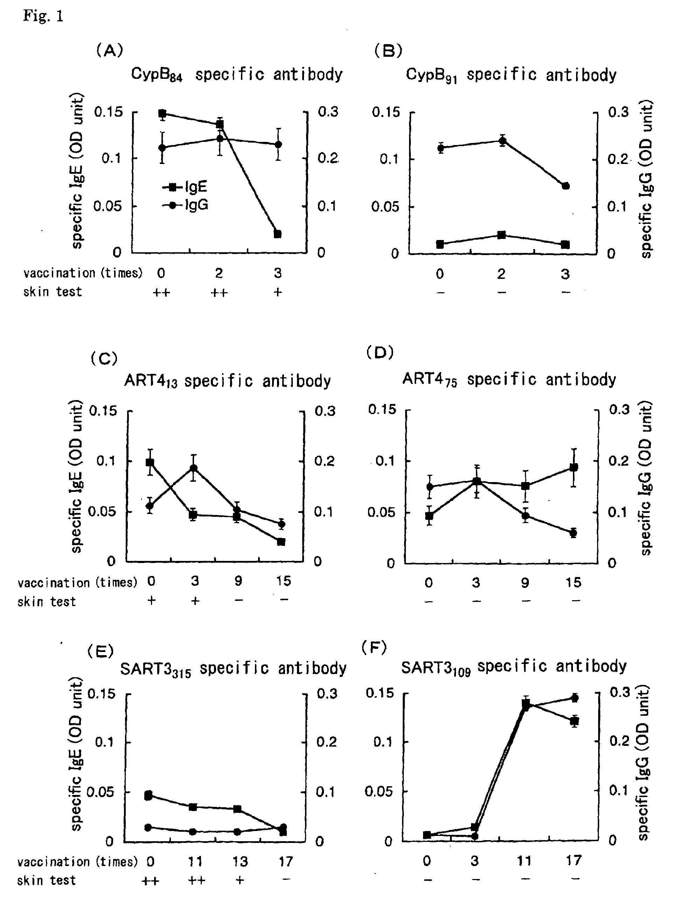

Fig. 1 illustrates that in a cancer vaccine therapy using a tumor rejection antigen-derived peptide, a type-I allergic reaction and the serum IgE level elicited by the tumor rejection antigen derived-peptide are suppressed by a non-allergic peptide having a partial sequence, different from the tumor rejection antigen-derived peptide, within an antigen molecule from which the tumor rejection antigen-derived peptide is derived. Figs. 1A and 1B illustrate the effect of administration of non-allergic peptide CypB₉₁ on type-I allergic reaction and serum IgE level against CypB₈₄ and CypB₉₁, respectively. Figs. 1C and 1D illustrate the effect of administration of non-allergic peptide ART4₇₅ on type-I allergic reaction and serum IgE level against ART4₁₃ and ART4₇₅, respectively. Figs. 1E and 1F illustrate the effect of administration of non-allergic peptide SART3₁₀₉ on type-I allergy and IgE level in serum against SART3₃₁₅ and SART3₁₀₉, respectively.

### DETAILED DESCRIPTION OF THE INVENTION

The present invention may be better illustrated hereinafter. It is understood that the following detailed description is explanatory only and is not restrictive of the present invention.

Technical and scientific terms used in the description have the meanings commonly understood by one of ordinary skill in the art to which the present invention pertains, unless otherwise defined. Publications and other materials referred in the description are incorporated herein by reference in their entireties as though set forth in full.

In the present invention, it was found that an allergic reaction elicited by a tumor rejection antigen-derived peptide that is an antigenic substance is suppressed by repeated administration of a peptide that is derived from the antigenic substance, which does not contain an epitope involved in eliciting the allergic reaction and contains an epitope that does not elicit the allergic reaction. It was also observed at this time that production of immunoglobulin E antibody (IgE) in serum is reduced in a living body in which the allergic reaction was elicited. 'An allergic reaction' in the present invention means type-I allergy mediated by IgE, hereafter.

The present invention was achieved based on the above findings and provides an agent for suppressing an allergic reaction that comprises a peptide derived from the same antigenic substance as the antigenic substance that elicits an allergic reaction. The above peptide has a property that it contains an epitope different from an epitope involved in eliciting the above allergic reaction, and does not elicit an allergic reaction. 'An epitope' herein means an antigenic determinant, i.e., a structural site that is a part of the antigen structure and is capable of inducing an immune response in a living body. In addition, a peptide containing an epitope is called 'an epitope peptide'. An epitope is recognized by each cell involved in an immune reaction, for example, by T cells through a T cell receptor, or by B cells through an antibody exhibiting on its surface, to induce an immune reaction. An immune reaction is classified into cellular immunity and humoral immunity, in each of which the cells involved are different. In addition, an induced immune reaction varies with the type of an epitope, including an epitope capable of inducing cellular immunity and that capable of inducing humoral immunity. Moreover, even if an epitope is capable of inducing humoral immunity, the type of antibody induced can vary. For example, an epitope peptide, which is restricted by major histocompatibility complex (MHC) class II and is recognized by CD4⁺ T cells, can enhance not only an action of T helper 1 cells (Th1), but also an action of T helper 2 cells (Th2) that are involved in IgE production, when it is administered to a living body. On the other hand, an epitope peptide restricted by MHC class I induces the clonal expansion of antigenic substance-specific CD8⁺ T cells, but does not induce the activation of Th2. Moreover, interferon γ produced by CD8⁺ T cells inhibits IgE response mediated by Th2. Therefore, it is more preferable that a peptide that does not cause the above allergic reaction has a property as an MHC class I-restricted one in addition to the above properties. Hereafter, a peptide containing an epitope that is involved in eliciting an allergic reaction may be called 'an allergic peptide' and a peptide containing an epitope that does not elicit an allergic reaction may be called 'a non-allergic peptide'.

The non-allergic peptide may be a peptide that can inhibit an action of an antigenic substance that can elicit an allergic reaction, and contains an epitope that is derived from the antigenic substance, which is different from an epitope eliciting an allergic reaction. There is no particular limitation to the amino acid sequence of the peptide. For a length of the peptide, a length that can generally exert antigenicity is sufficient, i.e., that consists of at least five amino acids, more preferably seven amino acids, and most preferably nine or ten amino acids. Although there is no particular limitation to the upper limit in the length of the peptide, it would be preferable that the peptide consists of nine to fifty amino acids or so, considering the efficiency of its action. Such a peptide can be obtained by designing and preparing peptides based on the amino acid sequence of an antigenic substance, which have amino acid sequences of a region not containing an epitope involved in the allergic reaction within the sequence of the antigenic substance, followed by selecting a peptide among them which is capable of inhibiting IgE production. With regard to the region in a sequence, a region in the neighborhood of the epitope peptide that can elicit the allergic reaction is preferably exemplified, and a region that partially overlaps with the epitope peptide can be the region. The peptides can be prepared by well-known methods from natural products, or by utilizing well-known methods such as a genetic engineering method and various synthesis methods. A peptide can be easily selected, for example, by carrying out an allergic skin test as described in Example 1 below, in which each peptide to be tested is intradermally injected and assessed by the size of an inflammatory flare that is evoked.

The antigenic substances can be foreign antigens, which have been known so far to elicit type-I allergic reaction, such as plant pollens, fungi, mites, animal smuts, house dusts, plant components, some drugs, and insect toxins. A self-antigen is included in the antigenic substances. A self-antigenic substance is exemplified by a self-antigen being detected in atopic dermatitis which can induce production of IgE [Susanne, N., FASEB J. (1998) 12: 1559-1569; Ulrich, A., Int. Arch. Allergy Immunol. (1999) 118: 193-196]; a tumor rejection antigen; human protein or peptide for medical use; or a self-antigen generated by destruction of autologous cells that were infected with a virus or the like by an immune reaction that is a host defense mechanism, and the like. Tumor rejection antigens are exemplified by cyclophilin B [J. Immunol. (1999) 163: 4994-5004] ; ART4 (Japan Patent Application Laid-open Pub. No. 2000-116383) ; SART2 (Japan Patent Application Laid-open Pub. No. Hei11-318455); and SART3 [Cancer Research (1999) 59: 4056-4063], but are not limited thereto. In addition, the antigenic substances can be living organisms per se, such as pollens, fungi, and mites, or can be cells, proteins, or peptides derived from organisms. Similarly, in case the antigenic substance is a self-antigen, the antigenic substance can be a cell that is a self-antigen, or a protein or peptide derived therefrom. In addition, the antigenic substance can be any of a natural product, a genetic engineering product, and a synthesis product, and there is no particular limitation so far as it elicits an allergic reaction.

Use of one or more of the above agents for suppressing an allergic reaction, for example, repeated administration to a living body, permits reduction of specific IgE production to an antigenic substance, and hence permits suppression of an IgE related type-I allergic reaction. Therefore, one or more of the above agents for suppressing an allergic reaction can be used for so-called desensitization as a desensitizer. Conventional desensitization has been carried out using a whole allergen, thereby subjecting patients to a heavy load. However, the above agent for suppressing an allergic reaction, which comprises a peptide that does not elicit an allergic reaction, is therefore safer and useful. In recent years, an antigenic substance-specific desensitization has been attempted with immunizing an MHC class II-restricted epitope peptide rather than immunizing a whole molecule of an antigenic substance. However, an MHC class II-restricted epitope peptide is capable of also activating Th2 as described above, thereby the MHC Class II-restricted epitope peptide could not always induce desensitization effectively. In addition, as described above, an MHC class I-restricted epitope peptide does not induce CD4⁺ T cells, and is also capable of inhibiting IgE production. Therefore, if the non-allergic peptide contained in the above agent for suppressing an allergic reaction is an MHC class I-restricted peptide, more effective and safer desensitization can be carried out than the conventional desensitization. Namely, an agent for suppressing an allergic reaction according to the present invention is useful as an agent for preventing and/or treating type-I allergic diseases, and can be used with a method for preventing and/or treating type-I allergic diseases. Type I allergic diseases are exemplified by the immediate hypersensitivity elicited by various foreign antigens or self-antigens such as asthma, including atopic asthma, dermatitis, including atopic dermatitis, hay fever, urticaria, anaphylaxis, pollenosis, and the like. In addition, it is well known that autoimmune diseases are caused by an immune reaction against self-antigens. Therefore, the above agent for suppressing an allergic reaction is useful also for treating autoimmune diseases in which an IgE antibody produced by self-antigens is involved.

As a concrete example of the above agent for suppressing an allergic reaction, in a case where the antigenic substance that can elicit an allergic reaction is a tumor rejection antigen derived-peptide capable of inducing and/or activating cytotoxic T cells, a non-allergic peptide is exemplified which is derived from the tumor rejection antigen having a partial sequence different from the tumor rejection antigen derived-peptide. For another example, in the case of an allergic peptide derived from a tumor rejection antigen is a peptide consisting of the 84^{th} to the 92^{nd} amino acids (CypB₈₄, KFHRVIKDF) (SEQ ID NO: 1 of the sequence listing) derived from cyclophilin B, the agent for suppressing an allergic reaction which comprises a peptide consisting of the 91^{st} to the 99^{th} amino acids (CypB₉₁, DFMIQGGDF) (SEQ ID NO: 2 of the sequence listing) derived from cyclophilin B, or a peptide (CypB₉₁-2F-Y) (SEQ ID NO: 9 of the sequence listing) obtained by replacing phenylalanine at the 2^{nd} position of CypB₉₁ with tyrosine, can be used. In addition, the above agent for suppressing an allergic reaction is exemplified by a peptide consisting of the 75^{th} to the 84^{th} amino acids (ART4₇₅, DYPSLSATDI) (SEQ ID NO: 4 of the sequence listing) derived from ART4 in case the allergic peptide derived from the tumor rejection antigen is a peptide consisting of the 13^{th} to the 20^{th} amino acids (ART4₁₃, AFLRHAAL) (SEQ ID NO: 3 of the sequence listing) derived from ART4; a peptide consisting of the 161^{st} to the 169^{th} amino acids (SART2₁₆₁, AYDFLYNYL) (SEQ ID NO: 5 of the sequence listing) derived from SART2 in case the allergic peptide derived from the tumor rejection antigen is a peptide consisting of the 899^{th} to the 907^{th} amino acids (SART2₈₉₉, SYTRLFLIL) (SEQ ID NO: 6 of the sequence listing) derived from SART2; and a peptide consisting of the 315^{th} to the 323^{rd} amino acids (SART3₃₁₅, AYIDFEMKI) (SEQ ID NO: 8 of the sequence listing) derived from SART3 in case the allergic peptide derived from the tumor rejection antigen is a peptide consisting of the 109^{th} to the 118^{th} amino acids (SART3₁₀₉, VYDYNCHVDL) (SEQ ID NO: 7 of the sequence listing) derived from SART3.

The above agent for suppressing an allergic reaction according to the present invention is not limited to the above examples. Any agent can be such an agent for suppressing an allergic reaction, so far as it is capable of inhibiting the action of an antigenic substance that elicits an allergic reaction and it comprises a non-allergic peptide derived from the antigenic substance containing an epitope different from an epitope involved in eliciting the allergic reaction. For example, in the case of a peptide derived from a human protein for medical use that elicits an allergic reaction, an agent for suppressing an allergic reaction can be obtained by preparing peptides having an amino acid sequence of a region not containing the epitope that is involved in eliciting the allergic reaction based on the protein from which the allergic peptide is derived, followed by selecting a peptide capable of inhibiting IgE production among them. In addition, also in the case of a human protein for medical use that elicits an allergic reaction, an agent for suppressing an allergic reaction can be obtained similarly as described above, by specifying the epitope that is involved in eliciting the allergic reaction in the protein, or an epitope peptide containing the epitope. The peptide for medical use that is derived from a human protein, or the human protein for medical use can be any protein or any peptide such as synthetic products, natural products, genetic engineering products, and it is not limited thereto. The peptide that elicits an allergic reaction according to the present invention is long enough so that the peptide can exert antigenicity, consisting of at least five amino acids, more preferably seven amino acids or more, most preferably nine or ten amino acids. There is no upper limitation to the length of the peptide. For example, in the case of a peptide for medical use that is used as a vaccine, it is preferable that the peptide consists of nine to fifty amino acids or so, in view of the efficiency of the action.

The above agent for suppressing an allergic reaction can be an agent that comprises one non-allergic peptide derived from one antigenic substance, or can be an agent that comprises plural non-allergic peptides derived from one antigenic substance, or can be an agent that comprises plural non-allergic peptides derived from plural antigenic substances. In a case where an allergic reaction is elicited against plural antigenic substances, it is preferable to use an agent for suppressing an allergic reaction that comprises plural non-allergic peptides derived from each antigenic substance. For example, in a case where the above tumor rejection antigen-derived allergic peptides are used in combination as peptides for medical use, it is preferable to use an agent for suppressing an allergic reaction that comprises non-allergic peptides derived from each tumor rejection antigen in the combination.

The above agent for suppressing an allergic reaction would be administered when the allergic reaction is elicited by an antigenic substance, or before, or when a human protein or a human protein-derived peptide for medical use which can elicit the allergic reaction, is administered. The amount of administration thereof can be determined depending on the degree of seriousness of the allergic reaction, or on the amount of the administrated protein or protein-derived peptide that elicits the allergic reaction. Generally, the amount is that capable of exerting antigenicity, for example, the amount ranges from 0.01 mg to 100 mg/day/adult human body, preferably from 0. 1 mg to 10 mg/day/adult human body. Administration can be carried out according to well-known methods for administrating a peptide for medical use, either systemically or locally, preferably intradermally or intramuscularly. When administering, the agent for suppressing an allergic reaction can be used solely, or in the presence or absence of, an appropriate adjuvant with or without linking such to a carrier. Any carriers can be used as long as it is not harmful to the human body and can enhance the antigenicity, such as cellulose, a polymerized amino acid, albumin, and so on. An adjuvant generally used for the peptide vaccination can be used for the present invention, such as Freund's incomplete adjuvant (FIA), aluminum adjuvant (ALUM), Bordetella pertussis vaccine, mineral oil, and so on.

The above agent for suppressing an allergic reaction can be used solely as a medicament, or can be prepared as a pharmaceutical composition in combination with a peptide or protein for medical use that elicits an allergic reaction.

For example, the above-exemplified peptide derived from a tumor rejection antigen can induce and/or activate tumor-specific cytotoxic T cells, so that it can be used as a peptide cancer vaccine. However, an allergic peptide among the peptides derived from a tumor rejection antigen elicits an allergic reaction in vivo, hence it cannot be used as a cancer vaccine. In such a case, an allergic reaction as a side effect is suppressed by using a non-allergic peptide derived from the same antigen as that in which a tumor rejection antigen-derived peptide is originated, so that even the tumor rejection antigen-derived allergic peptide can be used as a cancer vaccine. For example, a pharmaceutical composition is useful as a cancer vaccine, wherein the composition comprises a tumor rejection antigen-derived peptide that can induce and/or activate cytotoxic T cells but elicits an allergic reaction, and a peptide that is derived from the same antigen as the tumor rejection antigen-derived peptide, which epitope is different from the epitope contained in the tumor rejection antigen-derived peptide, and can suppress the allergic reaction. Such pharmaceutical compositions are exemplified by cancer vaccines comprising two peptides in combination as described below, but are not limited thereto: CypB₈₄ (SEQ ID NO: 1 of the sequence listing) and CypB₉₁ (SEQ ID NO: 2 of the sequence listing); CypB₈₄ (SEQ ID NO: 1 of the sequence listing) and CypB₉₁-2F-Y (SEQ ID NO: 9 of the sequence listing); ART4₁₃ (SEQ ID NO: 3 of the sequence listing) and ART4₇₅ (SEQ ID NO: 4 of the sequence listing); SART2₈₉₉ (SEQ ID NO: 6 of the sequence listing) and SART2₁₆₁ (SEQ ID NO: 5 of the sequence listing); and SART3₁₀₉ (SEQ ID NO: 7 of the sequence listing) and SART3₃₁₅ (SEQ ID NO: 8 of the sequence listing). A cancer vaccine comprising the above agent for suppressing an allergic reaction can be used solely, or in combination, or in the presence or absence of an appropriate well-known adjuvant, with or without linking such to a carrier. The above-mentioned carriers and adjuvants are used also for this purpose. The above cancer vaccine is useful as a medicament for treating cancer, and can be used with a method for preventing and/or treating cancer. Administration can be carried out utilizing well-known methods for administrating a peptide vaccine, either systemically or locally, and preferably intramuscularly. The amount of administration thereof can be determined depending on the degree of the recognition of the peptide by cytotoxic T cells. Generally, as an active ingredient, the amount ranges from 0.01 mg to 100 mg/day/adult human body, preferably from 0.1 mg to 10 mg/day/adult human body. Such an amount is administered from once every several days to once every several months.

It is preferable that the agent for suppressing an allergic reaction, the pharmaceutical composition, the cancer vaccine, or the medicament for treating cancer, according to the present invention, is formulated together with a suitable pharmaceutical carrier. Such a formulation comprises the above non-allergic peptides in an effective amount for treatment, peptides or proteins for medical use that elicit an allergic reaction, peptides derived from a tumor rejection antigen, or cancer vaccines, and additionally, pharmaceutically acceptable carriers and vehicles. Such carriers include physiological saline, buffered physiological saline, dextrose, water, glycerol, ethanol, and a mixture thereof, but are not limited thereto. A formulation may be selected according to the route of administration, and such a formulation is well known to persons skilled in the art. The agent for suppressing an allergic reaction, the pharmaceutical composition, the cancer vaccine, or the medicament for treating cancer, can be used solely or together with other compound(s) or pharmaceutical(s) necessary for treatment.

### Examples

Although the present invention is described in more detail by the examples shown below, the present invention is not limited to these examples.

### Example 1

In a skin test by pre-vaccination before phase I clinical trial to examine the effectiveness for cancer treatment of a peptide derived from cyclophilin B (CypB) and a peptide derived from ART4, which were identified as tumor rejection antigens capable of inducing and/or activating HLA-A24-restricted cytotoxic T cells, a peptide consisting of the 84^{th} to the 92^{nd} amino acids (Cyp₈₄) (KFHRVIKDF) (SEQ ID NO: 1 of the sequence listing) of CypB, and CypB₈₄F-Y that is a peptide obtained by partially modifying it [phenylalanine (F) at the 2^{nd} position was replaced with tyrosine (Y)], elicited immediate hypersensitivity skin reactions in eight of ten pre-vaccinated lung-cancer patients. Amino acid sequences of these two peptides are also well conserved in foreign organisms such as fungi and mites that can be allergens, and thus involvement of a foreign antigen was also indicated. However, a peptide consisting of the 13^{th} to the 20^{th} amino acids (ART4₁₃) (AFLRHAAL) (SEQ ID NO: 3 of the sequence listing) of ART4, which has no cross-reactivity to foreign antigens, also elicited an allergic skin reaction in ovarian cancer patients. Thus, it was determined that a type-I allergic reaction initiated by the above peptide vaccine was not elicited by a foreign antigen but was elicited by a self-antigen.

Therefore, it was investigated whether an allergic reaction to a peptide derived from the above self-antigen would also be observed in healthy volunteers. Skin tests were carried out with respect to self-derived tumor rejection antigen peptides capable of inducing cytotoxic T cells for 42 cases of healthy volunteers whose age ranges from 19 years old to 54 years old (mean age 29.2 years).

The following peptides used were prepared under the Good Manufacturing Practice by Multiple Peptide System Co.: a peptide consisting of the 84th to the 92nd amino acids (CypB₈₄) (KFHRVIKDF) (SEQ ID NO: 1 of the sequence listing) of CypB, a peptide consisting of the 91st to the 99th amino acids (CypB₉₁) (DFMIQGGDF) (SEQ ID NO: 2 of the sequence listing) of CypB, and a peptide (CypB₉₁-2F-Y) (SEQ ID NO: 9 of the sequence listing) obtained by replacing phenylalanine at the 2^{nd} position of CypB₉₁ with tyrosine; a peptide consisting of the 13^{th} to the 20^{th} amino acids (ART4₁₃) (AFLRHAAL) (SEQ ID NO: 3 of the sequence listing) and a peptide consisting of the 75^{th} to the 84^{th} amino acids (ART4₇₅) (DYPSLSATDI) (SEQ ID NO: 4 of the sequence listing) of ART4; a peptide consisting of the 161^{st} to the 169^{th} amino acids (SART2₁₆₁) (AYDFLYNYL) (SEQ ID NO: 5 of the sequence listing) and a peptide consisting of the 899^{th} to the 907^{th} amino acids (SART2₈₉₉) (SYTRLFLIL) (SEQ ID NO: 6 of the sequence listing) of SART2; and a peptide consisting of the 109^{th} to the 118^{th} amino acids (SART3₁₀₉) (VYDYNCHVDL) (SEQ ID NO: 7 of the sequence listing) and a peptide consisting of the 315^{th} to the 323^{rd} amino acids (SART3₃₁₅) (AYIDFEMKI) (SEQ ID NO: 8 of the sequence listing) of SART3. Each of these peptides can induce and/or activate cytotoxic T cells in an HLA-A24-restricted manner. In addition, purity of these peptides was 90% or higher, as assessed by reversed phase high-pressure liquid chromatography analysis. Each peptide was dissolved in DMSO, stored at -80°C, and diluted with physiological saline immediately before use.

For testing an allergic skin reaction, 10 µg of each peptide (50 µl volume) was intradermally injected, and evaluation was carried out 15 minutes after the injection. Results of skin tests were defined by the size of inflammatory flares as described below. The results are summarized in Tables 1 to 4, wherein 'NT' and 'S. T' mean 'not tested' and 'skin test' , respectively.
- : longest diameter (D) of flare < 10mm
+: 10mm ≦ D < 30mm
++: 30mm ≦ D < 50mm
+++: 50mm≦ D

### (Blank)

**Table 1**

| Healty donors no. | HLA-A | HLA-DR | CypB₈₄ | | | CypB₉₁ | | |
|---|---|---|---|---|---|---|---|---|
| | | | S. T | IgE | IgG | S. T | IgE | IgG |
| HD 1 | 24/26 | 1/6 | ++ | 0.048 | - | - | - | - |
| HD 2 | 24/33 | 2/9 | + | 0.155 | - | - | 0.077 - | - |
| HD 3 | 24 | 6/9 | ++ | - | - | - | - | 0.099 |
| HD 4 | 2402/0201 | 2 | ++ | - | - | - | - | - |
| HD 5 | 24 | 4/9 | - | - | 0.39 | - | - | 0.27 |
| HD 6 | 11/31 | 2/8 | ++ | 0.215 | - | - | - | - |
| HD 7 | 24/26 | 2 | ++ | - | - | - | - | - |
| HD 8 | 24 | 1/4 | + | - | - | - | - | - |
| HD 9 | 0201/0206 | 4/9 | + | - | - | - | - | - |
| HD 10 | 24/2 | 4/9 | + | - | - | - | - | - |
| HD 11 | 2 | 6/12 | ++ | - | - | - | + | - |
| HD 12 | 31/33 | 2/4 | ++ | - | - | - | - | - |
| HD 13 | 0201/0207 | 8/9 | + | 0.053 | - | - | 0. 069 | - |
| HD 14 | 0201/24 | 2/8 | + | 0.04 | - | - | - | - |
| HD 15 | 11/24 | 2 | ++ | - | 0. 044 | - | - | 0.045 |
| HD 16 | 24/33 | 1/6 | + | 0.138 | - | - | 0. 173 | - |
| HD 17 | 2409/2610 | 2 | + | - | - | - | - | - |
| HD 18 | 11/2409 | 2/6 | ++ | - | NT | - | - | NT |
| HD 19 | 2409/3319 | 2/9 | + | 0.027 | NT | - | - | NT |
| HD 20 | 2/2610 | 2/4 | + | 0.039 | - | - | - | - |
| HD 21 | 2409 | 4/11 | ++ | - | NT | - | 0.038 | NT |
| HD 22 | 11/24 | 1 | +++ | - | - | - | - | - |
| HD 23 | 2/31 | 2/12 | + | - | - | - | - | - |
| HD 24 | 2409 | 4/8 | +++ | - | - | - | - | - |
| HD 25 | 24/31 | 1/9 | ++ | - | - | - | 0.064 | - |
| HD 26 | 24/26 | 4 | + | 0.094 | - | - | - | - |
| HD 27 | 2/11 | 8/9 | + | - | - | - | - | - |
| HD 28 | 31 | 4 | + | - | - | - | - | 0.061 |
| HD 29 | 26/33 | 4/8 | +++ | - | NT | - | - | NT |
| HD 30 | 24 | 4/12 | ++ | 0.092 | - | - | 0.053 | - |
| HD 31 | 24/31 | 4 | + | - | NT | - | 0.714 | NT |
| HD 32 | 24/33 | 4/6 | + | - | - | - | - | - |
| HD 33 | 24 | NT | + | 0. 036 | 0. 777 | - | - | 1. 021 |
| HD 34 | 24 | NT | ++ | - | - | - | - | - |
| HD 35 | 24/31 | 4/8 | + | - | - | - | - | - |
| HD 36 | 24/26 | 2 | ++ | - | - | - | - | - |
| HD 37 | 2/24 | 2/4 | + | - | - | - | - | - |
| HD 38 | 11/24 | NT | - | - | 0. 089 | - | - | - |
| HD 39 | 2 | NT | + | 0.026 | - | - | - | - |
| HD 40 | 11/24 | NT | + | - | - | - | - | - |
| HD 41 | 2 | NT | + | - | - | - | - | - |
| HD 42 | 26/33 | NT | + | - | - | - | - | - |
| positive rate | | | 40/42 | 12/42 | 4/37 | 0/42 | 7/42 | 5/37 |

**Table 2**

| Healty donors no. | HLA-A | HLA-DR | ART4₁₃ | | | ART4₇₅ | | |
|---|---|---|---|---|---|---|---|---|
| | | | S. T | IgE | IgG | S. T | IgE | IgG |
| HD 1 | 24/26 | 1/6 | + | 0.047 | - | - | - | - |
| HD 2 | 24/33 | 2/9 | ++ | - | - | - | - | - |
| HD 3 | 24 | 6/9 | ++ | - | - | - | - | 0.162 |
| HD 4 | 2402/0201 | 2 | + | - | - | - | - | - |
| HD 5 | 24 | 4/9 | - | - | - | - | - | 0.118 |
| HD 6 | 11/31 | 2/8 | ++ | - | - | - | - | - |
| HD 7 | 24/26 | 2 | ++ | - | - | - | - | - |
| HD 8 | 24 | 1/4 | + | - | - | - | - | - |
| HD 9 | 0201/0206 | 4/9 | + | - | - | - | - | - |
| HD 10 | 24/2 | 4/9 | + | - | - | - | - | - |
| HD 11 | 2 | 6/12 | ++ | 0.112 | - | - | - | - |
| HD 12 | 31/33 | 2/4 | ++ | - | - | - | - | - |
| HD 13 | 0201/0207 | 8/9 | + | - | - | - | - | - |
| HD 14 | 0201/24 | 2/8 | NT | - | - | NT | - | - |
| HD 15 | 11/24 | 2 | + | - | 0.063 | - | - | 0.037 |
| HD 16 | 24/33 | 1/6 | + | - | - | - | - | - |
| HD 17 | 2409/2610 | 2 | ++ | - | - | - | - | - |
| HD 18 | 11/2409 | 2/6 | + | - | NT | - | - | NT |
| HD 19 | 2409/3319 | 2/9 | + | - | NT | - | - | NT |
| HD 20 | 2/2610 | 2/4 | + | - | - | + | - | - |
| HD 21 | 2409 | 4/11 | ++ | - | NT | - | - | NT |
| HD 22 | 11/24 | 1 | + | - | - | - | - | - |
| HD 23 | 2/31 | 2/12 | + | - | - | - | - | - |
| HD 24 | 2409 | 4/8 | + | - | - | - | - | - |
| HD 25 | 24/31 | 1/9 | + | 0.016 | - | - | NT | - |
| HD 26 | 24/26 | 4 | + | - | - | - | - | - |
| HD 27 | 2/11 | 8/9 | + | - | - | - | - | - |
| HD 28 | 31 | 4 | + | - | 0.053 | - | - | 0.052 |
| HD 29 | 26/33 | 4/8 | ++ | - | NT | - | - | NT |
| HD 30 | 24 | 4/12 | + | - | - | - | - | - |
| HD 31 | 24/31 | 4 | + | 0.502 | NT | - | NT | NT |
| HD 32 | 24/33 | 4/6 | + | - | - | - | - | - |
| HD 33 | 24 | NT | + | 0.044 | - | - | - | 0.395 |
| HD 34 | 24 | NT | ++ | - | - | - | - | - |
| HD 35 | 24/31 | 4/8 | + | - | - | - | - | 0. 054 |
| HD 36 | 24/26 | 2 | ++ | - | - | - | - | - |
| HD 37 | 2/24 | 2/4 | + | - | - | - | - | - |
| HD 38 | 11/24 | NT | - | - | - | - | - | - |
| HD 39 | 2 | NT | + | - | - | - | - | - |
| HD 40 | 11/24 | NT | + | - | - | - | - | - |
| HD 41 | 2 | NT | + | 0.014 | - | - | - | - |
| HD 42 | 26/33 | NT | ++ | - | - | - | - | - |
| positive rate | | | 39/41 | 6/42 | 2/37 | 1/41 | 0/40 | 6/37 |

**Table 3**

| Healty donors no. | HLA-A | HLA-DR | SART2₁₆₁ | | | SART2₈₉₉ | | |
|---|---|---|---|---|---|---|---|---|
| | | | S. T | IgE | IgG | S. T | IgE | IgG |
| HD 1 | 24/26 | 1/6 | - | - | - | - | - | - |
| HD 2 | 24/33 | 2/9 | - | NT | NT | ++ | NT | NT |
| HD 3 | 24 | 6/9 | - | - | - | + | - | - |
| HD 4 | 2402/0201 | 2 | - | - | - | + | - | - |
| HD 5 | 24 | 4/9 | - | - | - | ++ | - | 0.095 |
| HD 6 | 11 /31 | 2/8 | - | - | - | - | - | - |
| HD 7 | 24/26 | 2 | - | - | - | + | - | 0.024 |
| HD 8 | 24 | 1/4 | - | - | - | - | - | - |
| HD 9 | 0201/0206 | 4/9 | - | - | - | + | - | - |
| HD 10 | 24/2 | 4/9 | - | - | - | - | - | - |
| HD 11 | 2 | 6/12 | - | - | - | - | - | - |
| HD 12 | 31/33 | 2/4 | - | - | - | - | - | - |
| HD 13 | 0201/0207 | 8/9 | - | - | - | - | - | - |
| HD 14 | 0201/24 | 2/8 | - | - | - | NT | - | - |
| HD 15 | 11/24 | 2 | - | - | - | - | - | - |
| HD 16 | 24/33 | 1/6 | - | - | - | - | - | - |
| HD 17 | 2409/2610 | 2 | - | - | - | - | - | - |
| HD 18 | 11/2409 | 2/6 | - | - | - | + | - | - |
| HD 19 | 2409/3319 | 2/9 | - | - | + | - | - | 0.423 |
| HD 20 | 2/2610 | 2/4 | - | - | - | - | - | 0.036 |
| HD 21 | 2409 | 4/11 | - | - | 0.44 | - | - | 0. 316 |
| HD 22 | 11/24 | 1 | - | - | 0.196 | - | - | 0. 364 |
| HD 23 | 2/31 | 2/12 2 | - | NT | - | - | NT | - |
| HD 24 | 2409 | 4/8 | - | - | - | + | - | - |
| HD 25 | 24/31 | 1/9 | - | - | - | - | - | - |
| HD 26 | 24/26 | 4 | - | - | - | - | - | - |
| HD 27 | 2/11 | 8/9 | - | - | 0. 117 | - | - | - |
| HD 28 | 31 | 4 | - | - | - | - | - | - |
| HD 29 | 26/33 | 4/8 | - | - | - | - | - | - |
| HD 30 | 24 | 4/12 | - | 0.024 | - | - | - | - |
| HD 31 | 24/31 | 4 | - | NT | NT | - | NT | NT |
| HD 32 | 24/33 | 4/6 | - | - | - | - | - | - |
| HD 33 | 24 | NT | - | - | - | - | - | 0. 354 |
| HD 34 | 24 | NT | - | - | - | - | - | - |
| HD 35 | 24/31 | 4/8 | - | - | - | + | - | - |
| HD 36 | 24/26 | 2 | - | - | - | - | - | - |
| HD 37 | 2/24 | 2/4 | - | - | - | + | - | - |
| HD 38 | 11/24 | NT | - | - | - | - | - | - |
| HD 39 | 2 | NT | - | - | - | - | - | - |
| HD 40 | 11/24 | NT | - | - | - | - | - | - |
| HD 41 | 2 | NT | - | - | - | - | - | - |
| HD 42 | 26/33 | NT | - | - | - | - | - | - |
| positive rate | | | 0/42 | 0/39 | 3/40 | 10/41 | 0/39 | 7/40 |

**Table 4**

| Healty donors no. | HLA-A | HLA-DR | SART3₁₀₉ | | | SART3₃₁₅ | | |
|---|---|---|---|---|---|---|---|---|
| | | | S. T | IgE | IgG | S. T | IgE | IgG |
| HD 1 | 24/26 | 1/6 | - | - | - | - | - | - |
| HD 2 | 24/33 | 2/9 | ++ | 0.133 | - | - | - | - |
| HD 3 | 24 | 6/9 | - | - | 0.375 | - | - | - |
| HD 4 | 2402/0201 | 2 | - | - | - | - | - | - |
| HD 5 | 24 | 4/9 | - | - | 0.247 | - | - | 0.458 |
| HD 6 | 11/31 | 2/8 | - | - | - | - | - | - |
| HD 7 | 24/26 | 2 | + | - | - | + | - | - |
| HD 8 | 24 | 1/4 | - | - | - | - | - | - |
| HD 9 | 0201/0206 | 4/9 | - | - | - | - | - | - |
| HD 10 | 24/2 | 4/9 | + | 0.021 | - | - | - | - |
| HD 11 | 2 | 6/12 | - | - | - | - | 0.04 | - |
| HD 12 | 31/33 | 2/4 | - | - | - | - | - | - |
| HD 13 | 0201/0207 | 8/9 | - | 0.065 | - | - | 0.025 | - |
| HD 14 | 0201/24 | 2/8 | - | 0.034 | - | NT | - | - |
| HD 15 | 11/24 | 2 | - | 0.078 | 0.118 | - | - | 0.025 |
| HD 16 | 24/33 | 1/6 | - | 0. 157 | 0.025 | + | 0.115 | - |
| HD 17 | 2409/2610 | 2 | - | - | - | ++ | - | - |
| HD 18 | 11/2409 | 2/6 | - | 0.031 | NT | + | - | NT |
| HD 19 | 2409/3319 | 2/9 | - | - | NT | + | - | NT |
| HD 20 | 2/2610 | 2/4 | - | - | 0.072 | + | - | - |
| HD 21 | 2409 | 4/11 | - | 0.026 | NT | - | 0. 021 | NT |
| HD 22 | 11/24 | 1 | - | - | - | - | - | - |
| HD 23 | 2/31 | 2/12 | + | - | - | - | - | - |
| HD 24 | 2409 | 4/8 | - | - | - | - | - | - |
| HD 25 | 24/31 | 1/9 | - | 0.024 | - | - | 0.023 | - |
| HD 26 | 24/26 | 4 | - | - | - | - | - | - |
| HD 27 | 2/11 | 8/9 | - | 0.023 | - | + | - | - |
| HD 28 | 31 | 4 | - | - | 0.066 | - | 0.014 | 0.081 |
| HD 29 | 26/33 | 4/8 | - | - | NT | ++ | - | NT |
| HD 30 | 24 | 4/12 | - | - | - | +++ | - | - |
| HD 31 | 24/31 | 4 | - | 0.464 | NT | - | 0.039 | NT |
| HD 32 | 24/33 | 4/6 | - | - | - | + | - | - |
| HD 33 | 24 | NT | + | 0.071 | 0.865 | ++ | - | - |
| HD 34 | 24 | NT | - | - | - | ++ | - | - |
| HD 35 | 24/31 | 4/8 | - | - | 0.159 | ++ | - | 0.028 |
| HD 36 | 24/26 | 2 | + | - | - | ++ | - | - |
| HD 37 | 2/24 | 2/4 | + | - | - | ++ | - | - |
| HD 38 | 11/24 | NT | - | - | - | + | - | - |
| HD 39 | 2 | NT | - | - | - | - | - | - |
| HD 40 | 11/24 | NT | - | - | - | + | - | - |
| HD 41 | 2 | NT | - | - | - | + | - | - |
| HD 42 | 26/33 | NT | - | - | - | - | - | - |
| positive rate | | | 7/42 | 12/42 | 8/37 | 18/41 | 7/42 | 4/37 |

A positive skin reaction to CypB₈₄ peptide was observed in 40 cases among 42 cases of healthy volunteers (95%). ART4₁₃ also caused a positive reaction in a majority of healthy volunteers (39/41, 95%). SART2₈₉₉ caused a positive reaction in 10 cases among 41 cases (24%) . On the other hand, a positive reaction to CypB₉₁, ART4₇₅, or SART2₁₆₁, was scarcely observed in healthy volunteers (0/42, 1/41, or 0/42, respectively). With regard to SART3₃₁₅, 18 cases among 41 cases (44%) showed as being positive. With regard to SART3₁₀₉, 7 cases among 42 cases (17%) showed as being positive. The skin reactions were accompanied with flare and, in some cases, edema. The reaction developed within 1 minute and peaked at 5 to 20 minutes. Then, the reaction gradually diminished and disappeared after 1 hour, in most cases. These results indicate that the skin reactions elicited by the above peptides were typical type-I allergic reactions. In addition, a correlation was not found between these allergic skin reactions and the gender of the subjects, and between the reactions and HLA-A that is an MHC class I antigen, or between the reactions and HLA-DR serotype that is an MHC class II antigen.

Then, in order to assure that the above allergic skin reaction is mediated by IgE, levels of peptide-specific IgE and IgG in serum were measured. Levels of peptide-specific IgE and IgG in serum were measured by an enzyme-linked immunosorbent assay (ELISA). First, peptides were immobilized onto a 96-well flat bottom plate (Nunc Covalink, Fisher Scientific Co.) with disuccinimidylsuberate (PIERCE Co.) according to the manufactur's instructions. The plates, onto which each peptide (20 µg/well) was immobilized, was blocked with Block Ace (Yukijirushi Co.) and washed with phosphate-buffered saline containing 0.05% Tween 20 (Tween 20-PBS). Then, 100 µl/well of serum or plasma samples diluted with Block Ace containing 0.05% Tween 20 were added to the above plate. After incubation for two hours at 37 °C, the plates were washed with Tween 20-PBS and further incubated for 2 hours at 37°C together with 1:1000-diluted rabbit anti-human IgE antibody (ε chain-specific), anti-human IgG antibody (γ chain-specific) (DAKO Co. ), or anti-human IgG subclass-specific antibody (Zymed Laboratories Co.). The plates were washed 9 times, followed by the addition of 100 µl of 1:100-diluted goat anti-rabbit Ig-conjugated horseradish peroxidase dextran polymer (Envision, DAKO) to each well, and incubation at room temperature for 40 min. After washing, 100 µl of tetramethylbenzidine substrate solution (KPL Co.) was added, and then 1M phosphoric acid was added to stop the reaction. In order to estimate the peptide-specific IgE level, the optical density (OD) values of each sample were compared with those of serially diluted standard samples and expressed as OD unit/ml. Peptide-specific IgG levels were also calculated similarly, and the results were summarized in Tables 1 to 4. For the standard wells, 1:100-diluted anti-human IgE (Southern Biotechnology Associates Co.) or anti-human IgG (Leinco Co.) monoclonal antibodies were immobilized instead of the peptides, and the measurement was carried out in a manner similar to the above OD measurement. The total IgE level and the total IgG level of the samples were measured by SRL Co. at our request. In addition, it was assured that the IgE and IgG measured as above are the antibodies specific to each peptide, by such result as the addition of soluble peptides corresponding to each antibody to ELISA that is an assay system of each antibody resulted in the inhibition of the reaction accompanied with reduction of the absorbance, but addition of unrelated peptides did not lead to such inhibition.

Peptide-specific IgE to CypB₈₄ peptide and Peptide-specific IgE to CypB₉₁ peptide were detected in 12 cases and 7 cases, respectively, among 42 cases of healthy volunteers (Table 1) . On the other hand, IgG to CypB₈₄ peptide and IgG to CypB₉₁ peptide were detected in 4 cases and 5 cases, respectively, among 37 cases tested. Although the peptide-specific IgE was detected in the sera of 12 cases among 40 cases in which a skin reaction to CypB₈₄ was observed, there is no correlation between the degree of the skin reaction and the serum IgE level. However, in 2 cases (HD5 and HD38) in which skin reaction to CypB₈₄was not observed, a detectable level of IgG was observed in the sera, though the peptide-specific IgE was not observed.

Similarly, also with respect to ART4₁₃ and ART4₇₅ (Table 2) , SART2₁₆₁ and SART2₈₉₉ (Table 3) , as well as SART3₁₀₉ and SART3₃₁₅ (Table 4), the result of the above skin test was not always coincident with the production of the peptide-specific antibody. Such result may be caused by a low detection rate of serum IgE level. It is well known that most of the IgE produced in vivo is captured on the surface of a mast cell via high-affinity Fc receptors, so that only a very little amount of IgE is detected in the blood.

The allergic reaction and the specific IgE produced against an MHC class I-restricted and tumor rejection antigen-derived peptide as described above, which is derived from the self-antigen, have not been reported so far, and are disclosed here for the first time.

In addition, the subclass distribution of peptide-specific IgG and total IgG was analyzed in 6 cases among the above 42 cases. As a result, the major IgG subclass, in the total IgG, was IgG1 (59% to 79% of total IgG), with IgG2 being the second major subclass (17% to 29%). Relative contents of IgG3 and IgG4 were 8% to 3% and 6% to 2%, respectively. The subclass distribution of peptide-specific IgG to each of CypB₈₄, CyB₉₁, ART4₁₃, and ART4₇₅, was similar to that of the total IgG.

The phase I clinical trial of a peptide vaccine using CypB₉₁ was carried out on a lung cancer patient whose skin reaction and serum IgE level against CypB₈₄ were positive in the above allergic skin test. The skin reaction and serum IgE level against GypB₉₁ of the patient were negative. As illustrated in Fig. 1A, after 3 times administration of CypB₉₁, diminution of a skin reaction and a reduction of serum IgE level against CypB₈₄ were observed. At this time, no change was observed in the serum IgG level. A skin reaction and a serum IgG level against CypB₉₁ even after 3 times administration of CypB₉₁ were substantially the same as those before administration, i.e., negative (Fig. 1B). Similarly, inhibition of a skin reaction and a specific IgE level against CypB₈₄ by vaccination of CypB₉₁ or a peptide (CypB₉₁-2F-Y) (SEQ ID NO: 9 of the sequence listing) obtained by partially modifying the peptide were observed in 9 cases among 10 cases tested. These results revealed that the allergic reaction and the specific IgE level in serum against CypB₈₄ are reduced by administration of CypB₉₁.

The phase I clinical trial with ART4₇₅ was carried out on an ovarian cancer patient whose skin reaction against ART4₁₃ was positive. With respect to the patient, a skin reaction against ART4₇₅ was negative, and a specific IgE level in serum against ART4₁₃ and ART4₇₅ was low. As illustrated in Fig. 1C, reduction of the serum IgE level against ART4₁₃ was observed after 3 times administration of ART4₇₅, and disappearance of a skin reaction was observed after 9 times administration of the same, and the serum IgE level was further reduced after 15 times administration of the same. Although an ART4₇₅-specific IgE level in serum was gradually increased by administration of ART4₇₅, a skin reaction was negative both before and after administration of the same (Fig. 1D). Namely, an allergic reaction and a specific IgE level in serum against ART4₁₃ were reduced by administration of ART4₇₅.

The phase I clinical test using SART3₁₀₉ was carried out on a colon cancer patient whose skin reaction against SART3₃₁₅was positive. With respect to the patient, a skin reaction against SART3₁₀₉ was negative, and a specific IgE level in serum against SART3₃₁₅ and SART3₁₀₉ was low. As illustrated in Fig. 1E, reduction of a skin reaction and the serum IgE level against SART3₃₁₅ were observed after 13 times administration of SART3₁₀₉, and the skin reaction disappeared after 17 times administration of the same. Although a SART3₁₀₉-specific IgE level in serum was increased by administration of SART3₁₀₉, skin reactions before and after administration of the same were negative (Fig. IF). Namely, an allergic reaction and the specific IgE level in serum against SART3₃₁₅ were reduced by administration of SART3₁₀₉.

In addition, the phase I clinical trial with SART2₁₆₁ was carried out on a cancer patient whose skin reaction against SART2₈₉₉ was positive, diminution of a skin reaction and reduction of the serum IgE level against SART2₈₉₉ were observed after repeated administration of SART2₁₆₁, similar to the above 3 cases. Namely, an allergic reaction and the specific IgE level in serum against SART2₈₉₉ were reduced by administration of SART5₁₆₁.

These results reveal that a non-allergic peptide having a partial sequence, different from the tumor rejection antigen-derived peptide, within an antigen molecule from which the tumor rejection antigen-derived peptide is derived, could suppress an allergic reaction elicited by the tumor rejection antigen-derived peptide. Although allergic peptides such as GypB₈₄, ART4₇₄, and SART3₃₁₅ are useful as tumor rejection antigen peptides, they could not be used because of their side effects. However, it becomes possible to use these peptides as peptide vaccines for a patient in which the allergic skin reaction and the specific IgE level in serum against these peptides were reduced as described above. Such suppression of an allergic peptide-specific IgE reaction by vaccination with a non-allergic peptide that is derived from same antigen molecule is an extraordinary finding, which was also confirmed with combinations of other tumor rejection peptides.

### Example 2

IgE levels and IgG levels specific to the above MHC class I-restricted and tumor rejection antigen-derived peptides such as CypB₈₄, CypB₉₁, ART4₁₃, and ART4₇₅, which are derived from self-antigens, were measured in sera of atopic dermatitis (AD) patients. The measurement was carried out with a method similar to that described in Example 1. As a result, as summarized in Table 5, both levels of IgE and IgG specific to allergic peptides such as CypB₈₄ and ART4₁₃ were much higher than those of cancer patients or healthy volunteers. In addition, a pattern shows that serum IgE levels specific to non-allergic peptides such as CypB₉₁ and ART4₇₅ were remarkably different in comparison with that of cancer patients or healthy volunteers. This examination revealed that IgE to an MHC class I-restricted peptide derived from a self-antigen exists in serum also in atopic dermatitis.

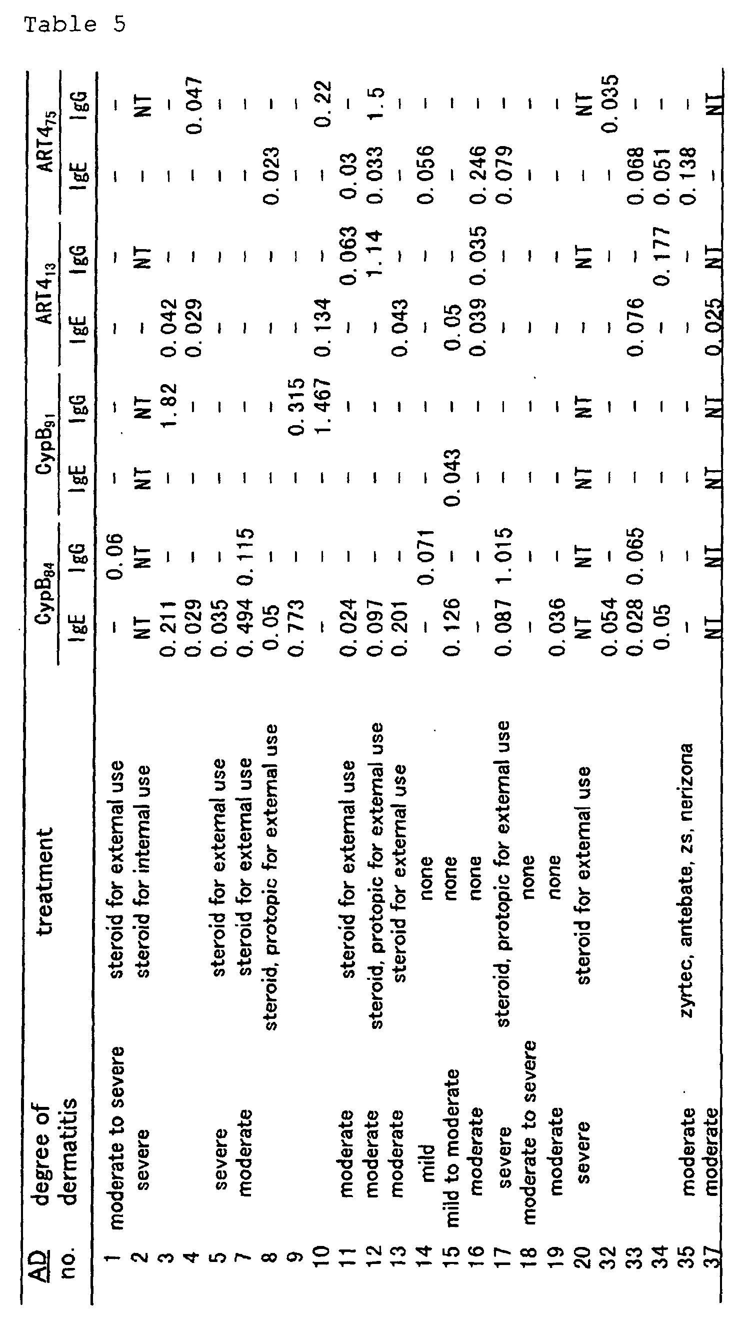

### INDUSTRIAL APPLICABILITY

According to the present invention, an agent for suppressing an allergic reaction can be provided, which can suppress the allergic reaction elicited by an antigenic substance, that comprises a non-allergic peptide derived from the antigenic substance and not containing an epitope involved in eliciting the allergic reaction. Using the agent for suppressing the allergic reaction permits the reduction of the IgE production induced by an antigenic substance, and hence suppressing the allergic reaction elicited by IgE. In addition, the agent for suppressing an allergic reaction can be used for desensitization of an antigenic substance that is an allergen. Namely, the agent for suppressing an allergic reaction according to the present invention is useful for preventing and/or treating type-I allergic diseases such as atopic dermatitis, allergic asthma, and pollenosis.

Further, the present invention in cancer vaccine therapy using a tumor rejection antigen-derived peptide permits suppressing a type-I allergic reaction that could be elicited by the tumor rejection antigen-derived peptide, by using a non-allergic MHC class I-restricted peptide derived from the same protein molecule as the tumor rejection antigen-derived peptide.

Moreover, the present invention is important for understanding allergic diseases, a host defense mechanism against tumor cells, and an action mechanism of a peptide cancer vaccine.

### Summary of the sequence listing

SEQ ID NO: 1: Peptide consisting of 9 amino acid residues from the 84^{th} residue to the 92^{nd} residue of cyclophillin B
SEQ ID NO: 2: Peptide consisting of 9 amino acid residues from the 91^{st} residue to the 99^{th} residue of cyclophillin B
SEQ ID NO: 3: Peptide consisting of 8 amino acid residues from the 13^{th} residue to the 20^{th} residue of ART4
SEQ ID NO: 4: Peptide consisting of 10 amino acid residues from the 75^{th} residue to the 84^{th} residue of ART4
SEQ ID NO: 5: Peptide consisting of 9 amino acid residues from the 161^{st} residue to the 169^{th} residue of SART2
SEQ ID NO: 6: Peptide consisting of 9 amino acid residues from the 899^{th} residue to the 907^{th} residue of SART2
SEQ ID NO: 7: Peptide consisting of 10 amino acid residues from the 109^{th} residue to the 118^{th} residue of SART3
SEQ ID NO: 8: Peptide consisting of 9 amino acid residues from the 315^{th} residue to the 323^{rd} residue of SART3
SEQ ID NO: 9: Designed peptide based on the peptide consisting of 9 amino acid residues from the 91^{st} residue to the 99^{th} residue of cyclophillin B

## Claims

1. An agent for suppressing an allergic reaction that comprises a peptide which is derived from the same antigenic substance as the antigenic substance eliciting the allergic reaction, and contains an epitope different from an epitope participating in elicitation of the allergic reaction and yet does not elicit the allergic reaction.

2. An agent for suppressing an allergic reaction that comprises a peptide which is derived from the same antigenic substance as the antigenic substance from which a peptide eliciting the allergic reaction is derived, and contains an epitope different from an epitope participating in elicitation of the allergic reaction and yet does not elicit the allergic reaction.

3. An agent for suppressing an allergic reaction that comprises a peptide which is derived from the same self-antigen as the self antigen eliciting the allergic reaction, and contains an epitope different from an epitope participating in elicitation of the allergic reaction and yet does not elicit the allergic reaction.

4. An agent for suppressing an allergic reaction that comprises a peptide which is derived from the same self-antigen as the self-antigen from which a peptide eliciting the allergic reaction is derived, and contains an epitope different from an epitope participating in elicitation of the allergic reaction and yet does not elicit the allergic reaction.

5. The agent for suppressing an allergic reaction according to claim 1, 2, 3 or 4, wherein the peptide which contains an epitope different from an epitope participating in elicitation of the allergic reaction and that does not elicit the allergic reaction is further an MHC class I-restricted one.

6. An agent for suppressing an allergic reaction elicited by a tumor rejection antigen-derived peptide capable of inducing a cytotoxic T lymphocyte, wherein the agent comprises a peptide which is derived from a tumor rejection antigen from which the tumor rejection antigen-derived peptide is derived, and contains an epitope different from an epitope participating in elicitation of the allergic reaction and yet does not elicit the allergic reaction.

7. The agent for suppressing an allergic reaction according to claim 6, wherein the tumor rejection antigen is one or more selected from a group consisting of cyclophilin B, ART4, SART2 and SART3.

8. An agent for suppressing an allergic reaction elicited by the peptide of SEQ ID NO: 1 shown in the sequence listing which is derived from cyclophilin B, wherein the agent comprises a peptide of SEQ ID NO: 2 or of SEQ ID NO: 9 shown in the sequence listing which is derived from cyclophilin B.

9. An agent for suppressing an allergic reaction elicited by the peptide of SEQ ID NO: 3 shown in the sequence listing which is derived from ART4, wherein the agent comprises a peptide of SEQ ID NO: 4 shown in the sequence listing which is derived from ART4.

10. An agent for suppressing an allergic reaction elicited by the peptide of SEQ ID NO: 6 shown in the sequence listing which is derived from SART2, wherein the agent comprises a peptide of SEQ ID NO: 5 shown in the sequence listing which is derived from SART2.

11. An agent for suppressing an allergic reaction elicited by the peptide of SEQ ID NO: 7 shown in the sequence listing which is derived from SART3, wherein the agent comprises a peptide of SEQ ID NO: 8 shown in the sequence listing which is derived from SART3.

12. An agent for preventing and/or treating type-I allergic diseases, comprising the agent for suppressing an allergic reaction according to any one of claims 1 to 11.

13. A method for preventing and/or treating type-I allergic diseases, comprising using the agent for suppressing an allergic reaction according to any one of claims 1 to 11.

14. A method for suppressing an allergy, comprising using the agent for suppressing an allergic reaction according to any one of claims 1 to 11.

15. A method for reducing production of immunoglobulin E antibody, comprising using the agent for suppressing an allergic reaction according to any one of claims 1 to 11.

16. A method of desensitization, comprising using the agent for suppressing an allergic reaction according to any one of claims 1 to 11.

17. A method for producing the agent for suppressing an allergic reaction according to any one of claims 1 to 11.

18. A pharmaceutical composition comprising a combination of a protein and/or peptide capable of eliciting an allergic reaction, and a peptide which is derived from the same antigenic substance as the protein and/or peptide and contains an epitope different from an epitope participating in elicitation of the allergic reaction and yet does not elicit the allergic reaction.

19. A cancer vaccine comprising a tumor rejection antigen-derived peptide having an activity to induce a cytotoxic T lymphocyte but eliciting an allergic reaction, and a peptide which is derived from the same antigenic substance as the tumor rejection antigen-derived peptide and contains an epitope different from an epitope contained in the tumor rejection antigen-derived peptide participating in elicitation of the allergic reaction and yet does not elicit the allergic reaction.

20. The cancer vaccine according to claim 19, comprising a peptide of SEQ ID NO: 1 shown in the sequence listing, and a peptide of SEQ ID NO: 2 or of SEQ ID NO: 9 shown in the sequence listing, wherein the peptides are derived from cyclophilin B.

21. The cancer vaccine according to claim 19, comprising a peptide of SEQ ID NO: 3 shown in the sequence listing, and a peptide of SEQ ID NO: 4 shown in the sequence listing, wherein the peptides are derived from ART4.

22. The cancer vaccine according to claim 19, comprising a peptide of SEQ ID NO: 6 shown in the sequence listing, and a peptide of SEQ ID NO: 5 shown in the sequence listing, wherein the peptides are derived from SART2.

23. The cancer vaccine according to claim 19, comprising a peptide of SEQ ID NO: 7 shown in the sequence listing, and a peptide of SEQ ID NO: 8 shown in the sequence listing, wherein the peptides are derived from SART3.

24. An agent for treating cancer, comprising the cancer vaccine according to any one of claims 19 to 23.

25. A method for preventing and/or treating cancer, comprising using the cancer vaccine according to any one of claims 19 to 23.

26. A method for producing the pharmaceutical composition according to claim 18.


SEQUENCE LISTING

<110>  ITOH, Kyogo

<120>  Desensitizers

<130>  GP02-1011/PCT

<150>  JP P2001-260046
<151>  2001-08-29

<160>  9

<170>  PatentIn version 3.1

<210>  1
<211>  9
<212>  PRT
<213>  Artificial

<220>
<223>  Peptide consisting of 9 amino acid residues from the 84th residue
       to the 92nd residue of cyclophillin B

<400>  1

Lys Phe His Arg Val Ile Lys Asp Phe
1               5


<210>  2
<211>  9
<212>  PRT
<213>  Artificial

<220>
<223>  Peptide consisting of 9 amino acid residues from the 91st residue
       to the 99th residue of cyclophillin B

<400>  2

Asp Phe Met Ile Gln Gly Gly Asp Phe
1               5


<210>  3
<211>  8
<212>  PRT
<213>  Artificial

<220>
<223>  Peptide consisting of 8 amino acid residues from the 13th residue
       to the 20th residue of ART4

<400>  3

Ala Phe Leu Arg His Ala Ala Leu
1               5


<210>  4
<211>  10
<212>  PRT

<213>  Artificial

<220>
<223>  Peptide consisting of 10 amino acid residues from the 75th residue
      to the 84th residue of ART4

<400>  4

Asp Tyr Pro Ser Leu Ser Ala Thr Asp Ile
1               5                   10


<210>  5
<211>  9
<212>  PRT
<213>  Artificial

<220>
<223>  Peptide consisting of 9 amino acid residues from the 161st residue
      to the 169th residue of SART2

<400>  5

Ala Tyr Asp Phe Leu Tyr Asn Tyr Leu
1               5


<210>  6
<211>  9
<212>  PRT
<213>  Artificial

<220>
<223>  Peptide consisting of 9 amino acid residues from the 899th residue
      to the 907th residue of SART2

<400>  6

Ser Tyr Thr Arg Leu Phe Leu Ile Leu
1               5


<210>  7
<211>  10
<212>  PRT
<213>  Artificial

<220>
<223>  Peptide consisting of 10 amino acid residues from the 109th residue
      to the 118th residue of SART3

<400>  7

Val Tyr Asp Tyr Asn Cys His Val Asp Leu
1               5                   10


<210>  8
<211>  9
<212>  PRT
<213>  Artificial

<220>

<223>  Peptide consisting of 9 amino acid residues from the 315th residue
      to the 323rd residue of SART3

<400>  8

Ala Tyr Ile Asp Phe Glu Met Lys Ile
1               5

<210>  9
<211>  9
<212>  PRT
<213>  Artificial

<220>
<223>  Designed peptide based on the peptide consisting of 9 amino acid
       residues from the 91st residue to the 99th residue of cyclophillin
       B

<400>  9

Asp Tyr Met Ile Gln Gly Gly Asp Phe
1               5